# EUROPEAN PATENT APPLICATION

(11) **EP 4 789 710 A1**
(43) Date of publication of application: **12.08.2026**
(21) Application number: 24874726.3
(22) Date of filing: 04.10.2024
(51) Int. Cl.: A61M 25/09, A61M 25/00

(54) **MEDICAL DEVICE**

(30) Priority: 06.10.2023 JP 2023174731
(71) Applicant: ASAHI INTECC CO., LTD., Seto-shi, Aichi, 489-0071 (JP)
(72) Inventor: SUGITA Rio, Seto-shi, Aichi 489-0071 (JP); NAKADA Masakazu, Seto-shi, Aichi 489-0071 (JP); NISHIGISHI Makoto, Seto-shi, Aichi 489-0071 (JP); FUJIWARA Soichiro, Seto-shi, Aichi 489-0071 (JP)
(74) Representative: Prinz & Partner mbB
(86) International application number: PCT/JP2024/035556
(87) International publication number: WO 2025/075126

(57) **Abstract**

A guide wire 10 (medical device) comprises: a long core 1; a cylindrical body 2 provided on the outer side of the core 1; a hydrophilic coat layer 61 formed of a first coating material and disposed on an outer peripheral surface of the cylindrical body 2; and a hydrophobic coat layer 62 formed of a second coating material and disposed on a radially outward side of the hydrophilic coat layer 61, wherein the hydrophilic coat layer 61 is capable of absorbing water. The hydrophilic coat layer 61 swells and is exposed on an outer surface of the hydrophobic coat layer 62 in a presence of a liquid. Such a guide wire 10 has good operability while having a simple structure.

## Description

### TECHNICAL FIELD

The present disclosure relates to a medical device.

### BACKGROUND ART

Medical devices such as guide wires have been conventionally used for guiding, to a target site, a catheter-like medical instrument that is inserted into a tubular organ of a human body, such as a blood vessel or a digestive organ. The guide wire is inserted into a lumen in the human body from the outer surface of the body, and is used by being inserted to a target site in the lumen in order to guide a combined device such as a catheter or an intracorporeal indwelling equipment to the target site. Since the body lumen from the outer surface of the body to the target site is often bent or branched, the guide wire is required to have a slidability that allows the guide wire to smoothly proceed in the lumen, and is also required to have a rotational followability that allows the guide wire to sensitively respond to the manipulation of the operator.

A guide wire is known in which the outer surface of the guide wire is coated with a hydrophilic resin that reduces the coefficient of dynamic friction, thereby improving the slidability and ensuring the rotational followability. For example, Patent Literature 1 discloses a guide wire including a metal core wire and a coil body in which a hydrophilic coat layer is formed on an outer surface of the guide wire.

### CITATION LIST

### Patent Literature

Patent Literature 1: Japanese Unexamined Patent Application Publication No. 2003-500116

### SUMMARY OF INVENTION

### TECHNICAL PROBLEM

When a hydrophilic coating layer is formed on the outer surface as in the guide wire described in Patent Literature 1, the outer surface of the guide wire becomes sticky when the guide wire is used in combination with a treatment device such as a catheter or an intracorporeal indwelling equipment at a position where the surrounding moisture decreases, for example, at a bent portion of a blood vessel. Since there is a concern that such stickiness of the outer surface affects operability, it is required to realize surface characteristics more excellent in operability with a simple structure.

The present disclosure has been made in view of the above circumstances, and an object of the present disclosure is to provide a medical device having good operability with a simple structure.

### SOLUTION TO PROBLEM

In order to achieve the above object, the present disclosure provides a medical device comprising: a long core; a cylindrical body provided on the outer side of the core; a hydrophilic coat layer formed of a first coating material and disposed on an outer peripheral surface of the cylindrical body; and a hydrophobic coat layer formed of a second coating material and disposed on a radially outward side of the hydrophilic coat layer, wherein the hydrophilic coat layer is capable of absorbing water (Disclosure 1).

According to this disclosure (Disclosure 1), since the hydrophobic coat layer is disposed on the outer side of the hydrophilic coat layer, the outer surface of the medical device is suppressed from being sticky even when the surrounding moisture decreases in the presence of the combined device or the like, and the coefficient of static friction decreases. On the other hand, since the hydrophilic coat layer is configured to be capable of absorbing water, a liquid reaches the inside of the hydrophobic coat layer from the outer surface, and the hydrophilic coat layer swells to improve the hydrophilicity of the outer surface of the medical device. Therefore, a medical device having good operability with a simple structure is realized.

In the above-described disclosure (Disclosure 1), an opening that communicates with an outer surface of the hydrophilic coat layer may be formed in the hydrophobic coat layer (Disclosure 2).

According to this disclosure (Disclosure 2), a liquid is supplied to the hydrophilic coat layer through the opening, and the hydrophilic coat layer swells. In addition, the hydrophilic coat layer swollen by the supplied liquid is exposed to the outside of the hydrophobic coat layer through the opening, thereby the hydrophilicity of the outer surface of the medical device is improved.

In the above-described disclosure (Disclosure 1 or 2), it is preferable that the hydrophilic coat layer swells and is exposed on an outer surface of the hydrophobic coat layer in a presence of a liquid (Disclosure 3).

In the above-described disclosure (Disclosure 1), the medical device may comprise an intermediate layer formed of the first coating material and the second coating material between the hydrophilic coat layer and the hydrophobic coat layer (Disclosure 4).

According to this disclosure (Disclosure 4), since the hydrophilic coat layer is configured to be capable of absorbing water, a liquid reaches the inside of the hydrophobic coat layer from the outer surface, and the intermediate layer containing a component of the first coating material that is a hydrophilic coating material and the hydrophilic coat layer swell to improve the hydrophilicity of the outer surface of the medical device. Therefore, a medical device having good operability with a simple structure is realized.

In the above-described disclosure (Disclosure 1 or 4), the second coating material may contain one or more polar solvents selected from a group consisting of lower chain alcohol, lower alkyl ethers, acetone, a halogen-containing solvent, and a lower alkylamine (Disclosure 5).

According to this disclosure (Disclosure 5), in the process of coating the hydrophobic coat layer onto the outer peripheral surface of the hydrophilic coat layer after coating the hydrophilic coat layer, the polar solvent contained in the hydrophobic coat layer partially dissolves the outer surface of the hydrophilic coat layer and the dissolved hydrophilic coat layer is mixed with a part of the hydrophobic coat layer, thereby the intermediate layer can be intentionally formed.

In the above-described disclosure (Disclosure 4 or 5), an opening that communicates with an outer surface of the intermediate layer may be formed in the hydrophobic coat layer (Disclosure 6).

According to this disclosure (Disclosure 6), a liquid is supplied to the intermediate layer through the opening, or is supplied to the hydrophilic coat layer via the intermediate layer, and the intermediate layer and the hydrophilic coat layer swell. In addition, the intermediate layer and the hydrophilic coat layer swollen by the supplied liquid are exposed to the outside of the hydrophobic coat layer through the opening, thereby the hydrophilicity of the outer surface of the medical device is improved.

In the above-described disclosure (Disclosure 4 or 5), it is preferable that at least one of the intermediate layer and the hydrophilic coat layer swells and is exposed on an outer surface of the hydrophobic coat layer in a presence of a liquid (Disclosure 7).

### EFFECT OF THE INVENTION

According to the present disclosure, it is possible to provide a medical device having excellent operability with a simple structure.

### BRIEF DESCRIPTION OF DRAWINGS

FIG. 1 is a longitudinal sectional view illustrating a structure of a guide wire according to an embodiment of the present disclosure.
FIG. 2 is a partially enlarged explanatory view illustrating a structure of a coating layer formed on the guide wire according to the embodiment.
FIG. 3 is a partially enlarged explanatory view illustrating a modified example of the structure of the coating layer.

### EMBODIMENTS OF THE INVENTION

A guide wire 10 according to an embodiment of the present disclosure will be explained below with reference to the drawings. The guide wire 10 is one example of a medical device according to the present disclosure and is used for inserting a combined device such as a catheter or an intracorporeal indwelling equipment into a blood vessel, a digestive organ, or the like. A distal end side of the guide wire 10 is a side to be inserted into a body, and a proximal end side of the guide wire 10 is a side to be operated by a professional such as a doctor. The present disclosure is not limited to the embodiment described below, and the described embodiment is merely an example for describing the technical feature of the present disclosure. The shapes and dimensions illustrated in the drawings are merely illustrated to facilitate understanding of the contents of the present disclosure, and do not accurately reflect actual shapes and dimensions. The present disclosure is also applicable to medical devices other than guide wires.

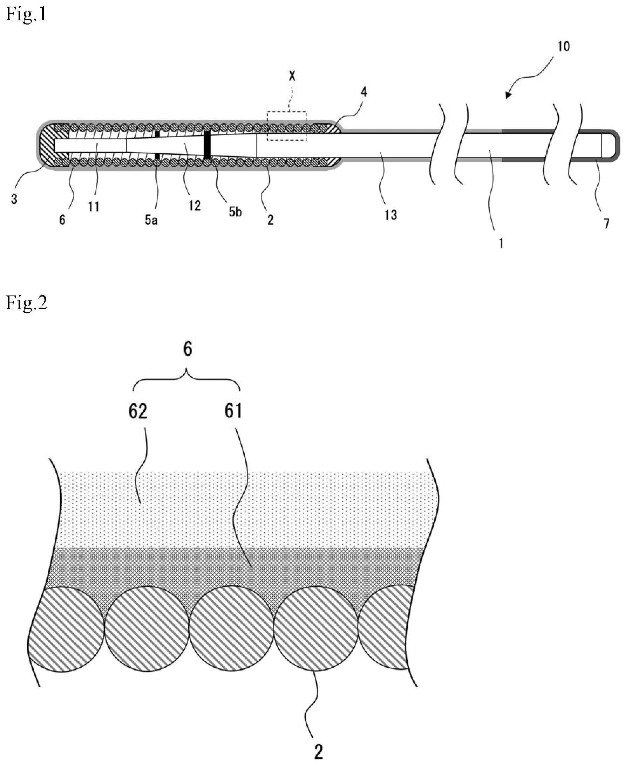

In the present description, the "distal end side" means a direction along an axial direction of the guide wire and a direction in which the guide wire proceeds toward a target site. The "proximal end side" means a direction along the axial direction of the guide wire and a direction opposite to the above-described distal end side. The "distal end" refers to an end portion on the distal end side of any member or site, and the "proximal end" refers to an end portion on the proximal end side of any member or site. The "distal end portion" refers to a part including the distal end of any member or site and extending from the distal end toward the proximal end side up to the middle of the member or the like, and the "proximal end portion" refers to a part including the proximal end of any member or site and extending from the proximal end toward the distal end side up to the middle of the member or the like. In FIG. 1, the left side in the drawing is the "distal end side" to be inserted into a body, and the right side in the drawing is the "proximal end side" to be operated by a professional.

FIG. 1 is a longitudinal sectional view illustrating a structure of a guide wire 10 according to the present embodiment. The guide wire 10 includes a long core 1 and a cylindrical body 2 provided on the outer side of the core 1. A distal tip 3 for joining the core 1 and the cylindrical body 2 is provided on the distal end of the guide wire 10, and a fixation portion 4 for fixing the core 1 and the cylindrical body 2 is provided on the proximal end of the cylindrical body 2.

The core 1 is an elongated member serving as a shaft of the guide wire 10. As illustrated in FIG. 1, the core 1 has a small diameter portion 11 on the distal end side and a large diameter portion 13 on the proximal end side, and has a tapered portion 12 disposed between the small diameter portion 11 and the large diameter portion 13 and having an outer diameter decreasing from the proximal end side toward the distal end side. The core 1 can be formed of, for example, materials such as stainless alloys (SUS302, SUS304, SUS316, and the like), superelastic alloys such as an Ni-Ti alloy, a piano wire, a nickel-chromium alloy, a cobalt alloy, and tungsten. However, the material for the core 1 is not limited to these materials. The core 1 may be formed of other known materials as long as the core 1 itself can be prevented from being cut and the distal end portion can be rotated.

The small diameter portion 11 has, for example, a flat shape (a substantially elliptical cross-sectional shape) obtained by pressing a columnar-shaped material. The large diameter portion 13 has a columnar shape in which the outer diameter is constant from the distal end to the proximal end. The tapered portion 12 has a circular truncated cone shape in which the outer diameter is gradually enlarged from the distal end toward the proximal end so as to connect the small diameter portion 11 and the large diameter portion 13.

The cylindrical body 2 is wound around the core 1 so as to cover the outer peripheries of the small diameter portion 11, the tapered portion 12, and a part of the large diameter portion 13 of the core 1. The cylindrical body 2 may be a single coil formed into a cylindrical shape by spirally winding one wire having a circular cross-section, or may be a hollow twisted wire coil formed into a cylindrical shape by a twisted wire that is obtained by twisting a plurality of wires. The cylindrical body 2 may be configured by combining a single coil and a hollow twisted wire coil. The cylindrical body 2 can be formed of, for example, stainless alloys (SUS302, SUS304, SUS316, and the like), superelastic alloys such as an Ni-Ti alloy, a piano wire, a nickel-chromium alloy, radiotransparent alloys such as a cobalt alloy, gold, platinum, tungsten, and radiopaque alloys such as an alloy containing these elements (for example, a platinum-nickel alloy). However, the material for the cylindrical body 2 is not limited to these materials. The cylindrical body 2 may be formed of known materials other than the above-described materials. In the present embodiment, the entire cylindrical body 2 is formed as a single member made of the same material, and the outer diameter thereof is configured to be constant from the distal end to the proximal end.

The distal tip 3 for joining the core 1 with the cylindrical body 2 is formed on the distal end of the guide wire 10 (i.e. the distal end of the core 1). The distal tip 3 is formed of a metal solder such as a silver solder, a gold solder, zinc, an Sn-Ag alloy, or an Au-Sn alloy, and the distal end of the core shaft 1 and the distal end of the cylindrical body 2 are fixed to each other by this metal solder. The distal tip 3 may be formed of an adhesive such as an epoxy adhesive so that the distal end of the core 1 and the distal end of the cylindrical body 2 are fixed to each other by the adhesive.

The fixation portion 4 for fixing the core 1 with the cylindrical body 2 is formed on the proximal end of the cylindrical body 2. The fixation portion 4 is formed of a metal solder such as silver solder, gold solder, zinc, an Sn-Ag alloy, and an Au-Sn alloy, and the proximal end of the cylindrical body 2 is fixed to the large diameter portion 13 of the core 1 by this metal solder. The fixation portion 4 may be formed of an adhesive such as an epoxy adhesive so that the large diameter portion 13 of the core 1 and the proximal end of the cylindrical body 2 are fixed to each other by the adhesive.

Two joint parts 5a and 5b for joining the tapered portion 12 of the core 1 with the cylindrical body 2 are formed inside the cylindrical body 2. The joint parts 5a and 5b are formed of a metal solder such as silver solder, gold solder, zinc, an Sn-Ag alloy, and an Au-Sn alloy, and the tapered portion 12 of the core 1 and the cylindrical body 2 are fixed to each other by this metal solder. The joint parts 5a and 5b may be formed of an adhesive such as an epoxy adhesive so that the tapered portion 12 of the core 1 and the cylindrical body 2 are fixed to each other by the adhesive.

A first coating layer 6 is formed on the outer peripheral surface of the guide wire 10 except for the proximal end portion of the large diameter portion 13 of the core 1, that is, on the outer peripheral surface from the distal tip 3 to the large diameter portion 13 of the core 1 through the cylindrical body 2 and the fixation portion 4. A second coating layer 7 different from the first coating layer 6 is formed on the outer peripheral surface of the proximal end portion of (the large diameter portion 13 of) the core 1.

The second coating layer 7 is formed of a hydrophobic resin material, and examples of the hydrophobic resin material include fluororesins such as polytetrafluoroethylene (PTFE), perfluoroalkoxyalkane (PFA), and FEP, and silicone. By coating the outer peripheral surface of the proximal end portion of the core 1 with such a hydrophobic resin material, the region where the first coating layer 6 is formed can be reduced, and the operability of the professional can be improved.

FIG. 2 is a partially enlarged explanatory view illustrating the structure of the first coating layer 6 formed on the guide wire 10, and illustrates the structure of a portion X in FIG. 1 in an enlarged manner. The first coating layer 6 has a structure in which a hydrophilic coat layer 61 disposed on the outer peripheral surface of the cylindrical body 2 and a hydrophobic coat layer 62 disposed on the radially outward side of the hydrophilic coat layer 61 are laminated.

The hydrophilic coat layer 61 is formed of a hydrophilic coating material (first coating material). Examples of the hydrophilic coating material may include a solution of a nonionic hydrophilic polymer such as polyvinyl alcohol, polyvinylpyrrolidone, polyethylene glycol, polyacrylamide, polymethylacrylamide, poly(2-hydroxyethyl methacrylate), or poly(N-hydroxyethylacrylamide), an anionic hydrophilic polymer such as polyacrylic acid, sodium polyacrylate, polymethacrylic acid, polymaleic acid, carboxymethyl cellulose, hyaluronic acid, or poly(2-acrylamido-2-methylpropanesulfonic acid), or a cationic hydrophilic polymer such as polyethyleneimine, polyallylamine, or polyvinylamine. The hydrophilic coat layer 61 can be formed by a known coating forming method, for example, by applying the above-described hydrophilic coating material to the outer peripheral surface from the distal tip 3 to the cylindrical body 2, the fixation portion 4, and the large diameter portion 13 of the core 1.

The hydrophobic coat layer 62 is formed of a hydrophobic coating material (second coating material). Examples of the hydrophobic coating material may include a silicone coating material such as a medical grade silicone solution, and a fluororesin such as silicone, polytetrafluoroethylene (PTFE), perfluoroalkoxyalkane (PFA), or FEP. The hydrophobic coat layer 62 can be formed by a known coating forming method such as applying the above-described hydrophobic coating material on the hydrophilic coat layer 61.

The hydrophobic coating material (second coating material) may contain one or more polar solvents selected from a group consisting of lower chain alcohol, lower alkyl ethers, acetone, a halogen-containing solvent, and a lower alkylamine. Examples of the lower chain alcohol may include a chain alkyl alcohol having 1 to 6 carbon atoms, and examples of the alkyl alcohol having 1 to 6 carbon atoms may include methanol, ethanol, n-propanol, i-propanol (IPA), n-butanol, sec-butanol, t-butanol, n-pentanol, and n-hexanol. Further, examples of the lower alkyl ethers include linear or cyclic alkyl ethers having 1 to 6 carbon atoms, and examples of the linear or cyclic alkyl ethers having 1 to 6 carbon atoms include dimethyl ether, diethyl ether, tetrahydrofuran, and 1,4-dioxane. Examples of the halogen-containing solvent include chain alkyl having 1 to 6 carbon atoms and containing 1 to 4 halogen atoms such as fluorine, bromine, and iodine, and examples of the chain alkyl having 1 to 6 carbon atoms and containing 1 to 4 halogen atoms include chloroform, dichloromethane, and carbon tetrachloride. Examples of the lower alkylamine include a chain alkylamine having 1 to 6 carbon atoms, and examples of the chain alkylamine having 1 to 6 carbon atoms include dimethylamine, methylamine, diethylamine, ethylamine, i-propylethylamine, and i-propylamine.

When the hydrophobic coat layer 62 is formed of, for example, a silicone coating material, the hydrophobic coat layer 62 is configured to be permeable to a liquid. Specifically, openings that communicate with the outer surface of the hydrophilic coat layer 61 are formed in the hydrophobic coat layer 62, and in the presence of a liquid, the hydrophilic coat layer 61 swells due to the liquid being supplied to the hydrophilic coat layer 61 through the openings. The hydrophilic coat layer 61 swollen by the supplied liquid is exposed to the outside of the hydrophobic coat layer 62 through the openings, thereby the hydrophilicity of the outer surface of the guide wire 10 is improved.

The state in which the hydrophobic coat layer 62 has openings may be realized, for example, by forming the hydrophobic coat layer 62 in a network form, by local gaps, cracks, perforations, or the like naturally formed in the process of forming the hydrophobic coat layer 62, or by forming the hydrophobic coat layer 62 by a known coating forming method and then subjecting the hydrophobic coat layer 62 to some surface processing.

According to such a guide wire 10, since the hydrophobic coat layer 62 is disposed on the outer side of the hydrophilic coat layer 61, the outer surface of the guide wire 10 is prevented from becoming sticky even when the surrounding moisture decreases, and the coefficient of static friction decreases. On the other hand, since the hydrophobic coat layer 62 is configured to be permeable to a liquid, the liquid reaches the inside of the hydrophobic coat layer 62 from the outer surface, and the hydrophilic coat layer 61 swells to improve the hydrophilicity of the outer surface of the guide wire 10. Therefore, the guide wire 10 having good operability is realized with a simple structure.

Originally, when a hydrophilic coat layer is disposed as the outermost layer of a medical device such as a guide wire, the coefficient of dynamic friction decreases, but the coefficient of static friction increases. On the other hand, when the hydrophobic coat layer is disposed as the outermost layer of a medical device such as a guide wire, the coefficient of dynamic friction increases, but the coefficient of static friction decreases. In the guide wire 10 of the present embodiment in which the hydrophobic coat layer 62 is disposed on the outer side of the hydrophilic coat layer 61, the presence of the hydrophobic coat layer 62 as the outermost layer has the effect of reducing the coefficient of static friction, and the swelling of the hydrophilic coat layer 61 to improve the hydrophilicity of the outer surface of the guide wire 10 (partial leaching of the hydrophilic coat layer 61 from the hydrophobic coat layer 62) has the effect of reducing the coefficient of dynamic friction. Therefore, a guide wire having two opposing characteristics and good operability is realized.

The guide wire according to the disclosure has been explained above with reference to the drawings, but the disclosure is not limited to the above embodiment and can be variously modified. For example, in the above embodiment, the case of the core 1 having the large diameter portion 13 on the proximal end side, the small diameter portion 11 on the distal end side, and the tapered portion 12 located between the large diameter portion 13 and the small diameter portion 11, has been explained as an example. However, the core used for the guide wire according to the disclosure may have an outer diameter that is constant from the proximal end side to the distal end side, or may have a structure in which the outer diameter changes in more stages.

An inner cylindrical body (coil body) shorter than the cylindrical body 2 may be disposed inside the cylindrical body 2 along the outer periphery of the core 1. For example, the inner cylindrical body may be arranged so that the inner cylindrical body is wound around the outside of the core 1 so as to cover the outer periphery of the core 1 from the small diameter portion 11 to a part of the tapered portion 12, and the cylindrical body 2 and the inner cylindrical body 8 overlap with each other on the outside of the core 1 only on the distal end portion of the guide wire 10.

The first coating layer 6 may be formed so as to cover at least the cylindrical body 2 provided on the outer side of the core 1, and may be disposed over the entire length of the cylindrical body 2, or may not be disposed over the entire length of the cylindrical body 2. The first coating layer 6 may not be directly formed on the outer surface of the cylindrical body 2, and for example, another coating layer may be formed between the cylindrical body 2 and the first coating layer 6. The second coating layer 7 formed on the outer surface of the proximal end portion of the guide wire 10 may be omitted, and for example, the entire outer surface of the guide wire 10 may be coated with the first coating layer 6.

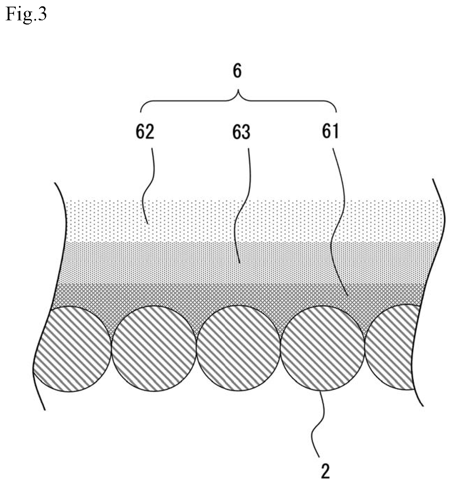

A modified example of the first coating layer 6 of the guide wire 10 explained in the above embodiments will be explained below with reference to FIG. 3.

In this modified example, the first coating layer 6 has a structure in which a hydrophilic coat layer 61 disposed on the outer peripheral surface of the cylindrical body 2, a hydrophobic coat layer 62 disposed on the radially outward side of the hydrophilic coat layer 61, and an intermediate layer 63 formed between the hydrophilic coat layer 61 and the hydrophobic coat layer 62 are laminated. The intermediate layer 63 is formed by mixing a hydrophilic coating material (first coating material) that forms the hydrophilic coat layer 61 and a hydrophobic coating material (second coating material) that forms the hydrophobic coat layer 62.

The hydrophilic coat layer 61 can be formed by a known coating forming method, for example, by applying the above-described hydrophilic coating material to the outer peripheral surface from the distal tip 3 to the cylindrical body 2, the fixation portion 4, and the large diameter portion 13 of the core 1. The hydrophobic coat layer 62 can be formed by a known coating forming method such as applying the above-described hydrophobic coating material on the hydrophilic coat layer 61. Here, when the silicone coating material for forming the hydrophobic coat layer 62 is applied to the upper layer of the hydrophilic coat layer 61, the alcohol component (IPA) contained in the silicone coating material dissolves the hydrophilic coating material on the surface layer side of the hydrophilic coat layer 61, and the intermediate layer 63 in which the silicone coating material and the hydrophilic coating material are mixed is formed.

Since the hydrophobic coat layer 62 is formed of a silicone coating material, the hydrophobic coat layer 62 is configured to be permeable to a liquid. Specifically, openings that communicate with the outer surface of the intermediate layer 63 are formed in the hydrophobic coat layer 62, and in the presence of a liquid, the intermediate layer 63 and the hydrophilic coat layer 61 swell due to the liquid being supplied to the intermediate layer 63 through the openings and to the hydrophilic coat layer 61 via the intermediate layer 63. The intermediate layer 63 and the hydrophilic coat layer 61 swollen by the supplied liquid are exposed to the outside of the hydrophobic coat layer 62 through the openings, thereby the hydrophilicity of the outer surface of the guide wire 10 is improved.

The state in which the hydrophobic coat layer 62 has openings may be realized, for example, by forming the hydrophobic coat layer 62 in a network form, by local gaps, cracks, perforations, or the like naturally formed in the process of forming the hydrophobic coat layer 62, or by forming the hydrophobic coat layer 62 by a known coating forming method and then subjecting the hydrophobic coat layer 62 to some surface processing.

According to such a guide wire 10 having the first coating layer 6 of such a three layer structure, since the hydrophobic coat layer 62 is disposed on the outer side of the hydrophilic coat layer 61 and the intermediate layer 63, the outer surface of the guide wire 10 is prevented from becoming sticky even when the surrounding moisture decreases, and the coefficient of static friction decreases. On the other hand, since the hydrophobic coat layer 62 is configured to be permeable to a liquid, the liquid reaches the inside of the hydrophobic coat layer 62 from the outer surface, and the intermediate layer 63 containing components of the hydrophilic coating material and the hydrophilic coat layer 61 swell to improve the hydrophilicity of the outer surface of the guide wire 10. Therefore, the guide wire 10 having good operability is realized with a simple structure.

Even in the guide wire 10 having the first coating layer 6 of a three layer structure, the presence of the hydrophobic coat layer 62 as the outermost layer has the effect of reducing the coefficient of static friction, and the swelling of the intermediate layer 63 and the hydrophilic coat layer 61 to improve the hydrophilicity of the outer surface of the guide wire 10 (partial leaching of the intermediate layer 63 and the hydrophilic coat layer 61 from the hydrophobic coat layer 62) has the effect of reducing the coefficient of dynamic friction. Therefore, a guide wire having two opposing characteristics and good operability is realized.

In particular, since the first coating layer 6 has a three layer structure, the durability of the hydrophobic coat layer 63 is improved. Further, even when the hydrophobic coat layer 63 disappears as a result of operation, since the intermediate layer 63 also contains components of the hydrophobic coating material, an increase in the coefficient of static friction can be suppressed.

## Claims

1. A medical device comprising: a long core;
a cylindrical body provided on the outer side of the core;
a hydrophilic coat layer formed of a first coating material and disposed on an outer peripheral surface of the cylindrical body; and
a hydrophobic coat layer formed of a second coating material and disposed on a radially outward side of the hydrophilic coat layer,
wherein the hydrophilic coat layer is capable of absorbing water.

2. The medical device according to claim 1, wherein an opening that communicates with an outer surface of the hydrophilic coat layer is formed in the hydrophobic coat layer.

3. The medical device according to claim 1 or 2, wherein the hydrophilic coat layer swells and is exposed on an outer surface of the hydrophobic coat layer in a presence of a liquid.

4. The medical device according to claim 1, comprising an intermediate layer formed of the first coating material and the second coating material between the hydrophilic coat layer and the hydrophobic coat layer.

5. The medical device according to claim 1 or 4, wherein the second coating material contains one or more polar solvents selected from a group consisting of lower chain alcohol, lower alkyl ethers, acetone, a halogen-containing solvent, and a lower alkylamine.

6. The medical device according to claim 4 or 5, wherein an opening that communicates with an outer surface of the intermediate layer is formed in the hydrophobic coat layer.

7. The medical device according to claim 4 or 5, wherein at least one of the intermediate layer and the hydrophilic coat layer swells and is exposed on an outer surface of the hydrophobic coat layer in a presence of a liquid.
